# EUROPÄISCHE PATENTSCHRIFT

(11) **EP 4 376 775 B1**
(45) Veröffentlichungstag und Bekanntmachung des Hinweises auf die Patenterteilung: **25.11.2026**
(21) Anmeldenummer: 22757493.6
(22) Anmeldetag: 20.07.2022
(51) Int. Cl.: A61F 2/844, A61F 2/86, A61L 27/00, A61F 2/82

(54) **IMPLANTAT, INSBESONDERE STENT, UND HERSTELLUNGSVERFAHREN**
IMPLANT, MORE PARTICULARLY STENT, AND METHOD OF PRODUCTION
IMPLANT, EN PARTICULIER STENT, ET PROCÉDÉ DE FABRICATION

(30) Priorität: 30.07.2021 DE 102021119828; 04.11.2021 DE 102021128698
(43) Veröffentlichungstag der Anmeldung: 05.06.2024
(73) Patentinhaber: Acandis GmbH, 75177 Pforzheim (DE)
(72) Erfinder: BÜCHERT, Michael, 75177 Pforzheim (DE); SCHÜSSLER, Andreas, 76327 Pfinztal (DE)
(74) Vertreter: Meissner Bolte Partnerschaft mbB
(86) Internationale Anmeldenummer: PCT/EP2022/070323
(87) Internationale Veröffentlichungsnummer: WO 2023/006537

(56) Entgegenhaltungen:
- DE-A1- 102019 121 562
- DE-A1- 102019 135 498

## Beschreibung

Die Erfindung betrifft ein medizinisches Implantat, insbesondere einen Stent, nach dem Oberbegriff des Patentanspruchs 1. Ferner betrifft die Erfindung ein Verfahren zum Herstellen eines solchen Implantats. Ein Implantat der eingangs genannten Art ist beispielsweise aus EP 1 362 564 B1 bekannt.

EP 1 362 565 B1 beschreibt einen selbstexpandierenden Stent, der insbesondere zur Behandlung von Aneurysmen geeignet ist. Konkret ist der Stent zur Abdeckung von Aneurysmen von Hirngefäßen einsetzbar.

Der bekannte Stent bzw. das bekannte Implantat umfasst eine rohrförmige Gitterstruktur, die sich selbsttätig von einem radial komprimierten Zustand in einen radial expandierten Zustand aufweiten kann. Die Gitterstruktur ist dabei aus mehreren miteinander verbundenen Gitterelementen gebildet, die Zellen der Gitterstruktur begrenzen.

Generell hat die gesamte Gitterstruktur eine Außenumfangsfläche und eine Innenumfangsfläche, wobei die Gitterelemente zwischen der Außenumfangsfläche und der Innenumfangsfläche angeordnet sind. Jedes Gitterelement hat für sich genommen jeweils eine Elementoberfläche, die mit der Außenumfangsfläche und der Innenumfangsfläche deckungsgleich ist. Die Oberflächen des jeweiligen Gitterelements, die sich zwischen der Außenumfangsfläche und der Innenumfangsfläche erstrecken, sind ebenfalls Bestandteil der Elementoberfläche. Jedes Gitterelement weist folglich eine Elementoberfläche auf, die sich um den gesamten Umfang des Gitterelements erstreckt.

Die DE 10 2019 135 498 A1 zeigt ein medizinisches System zur Behandlung von Stenosen mit einer röhrchenförmigen Gitterstruktur und einer Abdeckung, die den Außen- und/oder Innenumfang der röhrchenförmigen Gitterstruktur überspannt.

Stents der eingangs genannten Art haben den Nachteil, dass sie im eingesetzten Zustand den Blutstrom durch ein Gefäß beeinflussen, was einerseits zur Bildung von Blutgerinnseln führen kann und andererseits auch eine Integration des Stents in das natürliche Blutgefäß erschweren kann. Insofern wurden Weiterentwicklungen derartiger Stents getätigt, bei welchen die Stents mit antithrombogenen Substanzen beschichtet sind. Derartige Medikamenten-abgebende Stents haben die Gefahr der Bildung von Blutgerinnseln bzw. Thrombosen reduziert. Allerdings ist die Abgabe von antithrombogenen Substanzen zeitlich begrenzt, da sich der Wirkstoff von der Gitterstruktur ablöst, bis die Gitterstruktur im Wesentlichen wirkstofffrei ist.

Insofern besteht Bedarf an medizinischen Implantaten, die langfristig eine Bildung von Blutgerinnseln vermeiden und sich so besser in die organischen Strukturen integrieren.

Die Aufgabe der Erfindung besteht also darin, bisherige medizinische Implantate derart weiterzuentwickeln, dass deren Langzeitwirkungen verbessert sind. Ferner ist es Aufgabe der Erfindung, ein Herstellungsverfahren für ein derartiges medizinisches Implantat anzugeben.

Erfindungsgemäß wird diese Aufgabe im Hinblick auf das medizinische Implantat durch den Gegenstand des Patentanspruchs 1 gelöst. Im Hinblick auf das Herstellungsverfahren löst die Erfindung die obengenannte Aufgabe durch den Gegenstand des Patentanspruchs 14.

So beruht die Erfindung auf dem Gedanken, ein medizinisches Implantat, insbesondere einen Stent, mit einer rohrförmigen Gitterstruktur anzugeben, die von einem radial komprimierten Zustand in einen radial expandierten Zustand überführbar ist. Die Gitterstruktur weist Gitterelemente auf, die Zellen der Gitterstruktur begrenzen. Die Gitterelemente weisen außerdem eine Elementoberfläche auf. Erfindungsgemäß ist zur Vergrößerung der Elementoberfläche jedes Gitterelements eine Polymer-Nanostruktur vorgesehen, die vereinzelt auf der gesamten Elementoberfläche des Gitterelements verteilt anhaftet. Ferner ist eine antithrombogene Beschichtung vorgesehen, die sich über die mit der polymeren Nanostruktur vergrößerte Strukturoberfläche des Gitterelements erstreckt.

Der Vorteil der Erfindung besteht darin, dass durch die Vergrößerung der Elementoberfläche im Vergleich zum Stand der Technik deutlich mehr antithrombogene Substanz auf der Gitterstruktur abgelagert werden kann als dies bei Implantaten aus dem Stand der Technik möglich ist. Hierdurch können also größere Mengen an einem funktionalen Wirkstoff, insbesondere einem antithrombogenen Wirkstoff, auf der Gitterstruktur gelagert werden, so dass die Langzeitwirksamkeit des antithrombogenen Wirkstoffs verbessert wird.

Die Polymer-Nanostruktur vergrößert die Elementoberfläche der Gitterelemente.

Die vergrößerte Strukturoberfläche hat außerdem den Vorteil, dass sich durch die Strukturierung der Strukturoberfläche Endothelzellen besser anlagern bzw. verbessert neu bilden können, so dass sich das erfindungsgemäß medizinische Implantat sehr gut und schnell in die organische Umgebung integriert.

Die Polymer-Nanostruktur haftet nicht vollumfänglich auf der gesamten Elementoberfläche des Gitternetzes an. Vielmehr ist es vorgesehen, dass die Polymer-Nanostruktur nur vereinzelt bzw. punktuell mit der Elementoberfläche in Kontakt steht. Die Elementoberfläche im Sinne der vorliegenden Anmeldung ist also die Gesamtoberfläche jedes Gitterelements ohne die Polymer-Nanostruktur. Die Polymer-Nanostruktur wird dann auf die Elementoberfläche aufgebracht, wobei die Polymer-Nanostruktur punktuell an der Elementoberfläche anhaftet. Die daraus resultierende Gesamtoberfläche des Gitterelements, also die Kombination aus der Oberfläche der Polymer-Nanostruktur und den von der polymeren Nanostruktur freigelassenen Bereichen der Elementoberfläche, bildet die Strukturoberfläche des Gitterelements. Vereinfacht bezeichnet also die Elementoberfläche die Oberfläche des Gitterelements ohne Polymer-Nanostruktur, wogegen die Strukturoberfläche die Oberfläche des Gitterelements mit der Polymer-Nanostruktur bezeichnet.

Die Polymer-Nanostruktur kann aus resorbierbaren oder nicht-resorbierbaren Polymeren gebildet sein. Die Polymer-Nanostruktur kann insbesondere aus nicht-resorbierbaren Polymeren auf Polyurethanbasis hergestellt sein. Resorbierbare Biopolymere zur Bildung der Polymer-Nanostruktur können beispielsweise Polylactide (Poly-L-lactid (PLLA), Polylactid-co-Glycolid (PLGA)) oder Materialmischungen hiervon (Co-Polymere) umfassen. Weitere bevorzugte Materialien sind Polycaprolacton (PCL), Polylactid-co-caprolacton (PLCL), Poly-D-lactid (PDLA) oder Poly-DLlactid (PDLLA) sowie weitere Zusammensetzungen.

Bei einer bevorzugten Ausführungsform der Erfindung ist die Polymer-Nanostruktur hydrophob an die Elementoberfläche gebunden. Die hydrophobe Anbindung reduziert die Kontaktfläche zwischen der Polymer-Nanostruktur und der Elementoberfläche, so dass eine größere Oberfläche der Polymer-Nanostruktur für die Anlagerung von Endothelzellen und/oder der antithrombogenen Beschichtung zur Verfügung steht. Durch die hydrophobe Anlagerung wird also eine besonders hohe Vergrößerung der Strukturoberfläche erreicht.

Die antithrombogene Beschichtung kann zumindest teilweise in die Polymer-Nanostruktur eingebettet sein oder die Polymer-Nanostruktur umhüllen. Es ist auch möglich, dass die antithrombogene Beschichtung mit einer Oberfläche der Polymer-Nanostruktur zumindest teilweise vernetzt ist. Die Einbettung der antithrombogenen Beschichtung in die Polymer-Nanostruktur erhöht weiterhin die Langzeitbeständigkeit bzw. Langzeitwirksamkeit der antithrombogenen Beschichtung durch langsame Diffusion der Wirkstoffe an die Oberfläche.

Die Einbettung der antithrombogenen Beschichtung in die Polymer-Nanostruktur schützt die antithrombogene Beschichtung vor Abrieb, der beispielsweise durch das Zuführen des Implantats durch einen Katheter entstehen kann. Die antithrombogene Beschichtung ist damit am Behandlungsort länger wirksam. Ähnliches gilt für den Fall, dass die antithrombogene Beschichtung mit einer Oberfläche der Polymer-Nanostruktur zumindest teilweise vernetzt ist. Eine starke Bindung der antithrombogenen Beschichtung an die Polymer-Nanostruktur ist insoweit vorteilhaft.

Bei einer bevorzugten Ausführungsform der Erfindung weist die antithrombogene Beschichtung Fibrin und ein Antikoagulanz auf. Es ist auch möglich, dass die antithrombogene Beschichtung Fibrin und ein Antikoagulanz enthält.

Das Antikoagulanz kann insbesondere Heparin sein, das kovalent an das Fibrin gebunden ist. Die kovalente Bindung des Heparins an das Fibrin sorgt für eine besonders beständige antithrombogene Beschichtung. Dabei ist sowohl Fibrin, als auch Heparin antithrombogen wirksam. Die beiden Wirkstoffe ergänzen sich insoweit und erreichen damit eine besonders gute antithrombogene Wirkung.

Bei einer bevorzugten Ausführungsform der Erfindung ist vorgesehen, dass die Polymer-Nanostruktur durch Polymertröpfchen gebildet ist, die auf der Elementoberfläche des Gitterelements verteilt angelagert sind. So können sich beispielsweise auf einer Fläche von 16 µm² mindestens sechs Polymertröpfchen anlagern, wobei die Anlagerung vorzugsweise hydrophob erfolgt. Wenn jedes Polymertröpfchen in dieser Konstellation beispielsweise eine Fläche von ca. 0,66 µm² der Elementoberfläche abdeckt, wird diese abgedeckte Fläche auf eine lokale Strukturoberfläche von ca. 2,2 µm² vergrößert. Dies ergibt sich aus der nahezu kugelartigen Oberfläche der Polymertröpfchen. Eine Elementoberfläche von 16 µm² wird also mit sechs hydrophob angelagerten Tröpfchen auf eine Strukturoberfläche von 25,23 µm² vergrößert. Dies entspricht einer Oberflächenvergrößerung zwischen der Elementoberfläche und der Strukturoberfläche von 158%.

Bei einer bevorzugten Ausführungsform der Erfindung ist vorgesehen, dass die Polymertröpfchen jeweils einen Durchmesser von höchstens 1500 nm, insbesondere höchstens 1250 nm, insbesondere höchstens 1000 nm, insbesondere höchstens 750 nm, insbesondere höchstens 500 nm, insbesondere höchstens 400 nm, insbesondere höchstens 250 nm, insbesondere höchstens 100 nm, insbesondere höchstens 50 nm, aufweisen. Es ist möglich, dass die Polymertröpfchen unterschiedliche Durchmesser umfassen. Die zuvor genannten Durchmesserwerte bilden jedoch einen Durchschnitt der Durchmesser aller Polymertröpfchen. Üblicherweise bilden die Polymertröpfchen eine Kugeloberfläche, die nur in einem kleinen Segment unterbrochen ist, weil das Polymertröpfchen dort hydrophob an die Elementoberfläche gebunden ist. Je kleiner der Durchmesser der Polymertröpfchen ist, desto größer ist somit die vergrößerte Strukturoberfläche des Gitterelements, die sich aus der Oberfläche des Polymertröpfchens und der Elementoberfläche des Gitterelements zusammensetzt.

Generell bildet die Polymer-Nanostruktur vorzugsweise einen porösen Haftvermittler auf der Elementoberfläche des Gitterelements. Der Haftvermittler wird deshalb als porös bezeichnet, weil die Polymer-Nanostruktur nicht vollflächig auf der Elementoberfläche anhaftet, sondern lediglich stellenweise bzw. punktuell. Wenn die Polymer-Nanostruktur durch Polymertröpfchen gebildet ist, so liegen zwischen den einzelnen Tröpfchen freie Bereiche der Elementoberfläche des Gitterelements vor. Diese freien Bereiche sind auch Teil der Strukturoberfläche.

Es ist auch möglich, dass die Polymer-Nanostruktur durch ein Nanovlies aus Polymerfasern gebildet ist, das sich vollständig um das Gitterelement erstreckt. Das Nanovlies kann beispielsweise durch Elektrospinnen hergestellt sein.

Bei einer solchen Polymer-Nanostruktur sind einzelne Fasern chaotisch miteinander verknüpft bzw. kreuzen sich chaotisch und bilden insoweit ein Vlies. Das Vlies ummantelt die Elementoberfläche jedes Gitterelements. Da die einzelnen Fasern jedoch nur stellenweise bzw. punktuell mit der Elementoberfläche des Gitterelements in Kontakt stehen, wird auch eine solche Polymer-Nanostruktur im Rahmen der vorliegenden Anmeldung als poröser Haftvermittler bezeichnet.

Die antithrombogene Beschichtung, die sich über die Polymer-Nanostruktur erstreckt, kann sich insbesondere mit dem Nanovlies aus Polymerfasern verbinden. Insbesondere kann der antithrombogene Wirkstoff der antithrombogenen Beschichtung zumindest teilweise in das Nanovlies eingebettet sein. Dadurch ist eine besonders langzeitwirksame Wirkstoffabgabe erreicht, da das Nanovlies ähnlich wie ein Schwamm eine vergleichsweise große Menge der antithrombogenen Beschichtung aufnehmen kann.

Unabhängig von der Art der Polymer-Nanostruktur, insbesondere unabhängig davon, ob die Polymer-Nanostruktur aus Polymertröpfchen und/oder einem Nanovlies gebildet ist, wird mit der Polymer-Nanostruktur vorzugsweise erreicht, dass die Größe der Strukturoberfläche des Gitterelements um mindestens 150%, insbesondere mindestens 200%, insbesondere mindestens 250%, insbesondere mindestens 300%, insbesondere mindestens 400%, insbesondere mindestens 500%, gegenüber der Größe der Elementoberfläche vergrößert wird. Mit anderen Worten wird also die Oberfläche des Gitterelements, die zur Anlagerung der antithrombogenen Beschichtung zur Verfügung steht, durch die Aufbringung der Polymer-Nanostruktur zwischen die Elementoberflächen des Gitterelements und die antithrombogene Beschichtung eine erhebliche Vergrößerung der Oberfläche erreicht, an die sich die antithrombogene Beschichtung anbinden kann.

Es hat sich gezeigt, dass die Vergrößerung der Strukturoberfläche gegenüber der Elementoberfläche erheblichen Einfluss auf die Wirksamkeit des antithrombogenen Wirkstoffs, insbesondere in zeitlicher Hinsicht, hat. Besonders vorteilhaft wirkt sich die Vergrößerung der Strukturoberfläche im Hinblick auf die Integration des Implantats in das umgebende biologische Gewebe aus. Durch die Vergrößerung der Elementoberfläche, also der Oberfläche des blanken Gitterelements, mittels der Anlagerung der Polymer-Nanostruktur wird in der Praxis zusätzlich erreicht, dass sich Endothelzellen gut an die Gitterelemente anlagern können. Das führt zu einer besonders schnellen Endothelialisierung des Implantats, wodurch dieses sich gut in das natürliche Gewebe integriert und so Verwirbelungen, die später zu einer Bildung von Blutgerinnseln führen können, vermieden werden.

Die Elementoberfläche kann vorzugsweise oberflächenbehandelt, insbesondere elektropoliert oder mechanisch blank sein. Die Polymer-Nanostruktur haftet trotz der glatten bzw. blanken Elementoberfläche überraschenderweise dennoch gut an der Elementoberfläche an.

Bei einer weiteren bevorzugten Ausführungsform des erfindungsgemäßen Implantats weist das Gitterelement eine Kernschicht und eine Mantelschicht auf. Die Kernschicht kann ein röntgensichtbares Material, insbesondere Platin oder eine Platinlegierung, und die Mantelschicht und ein superelastisches Material, insbesondere eine Nickel-Titan-Legierung, aufweisen oder daraus bestehen.

Im Wesentlichen kann das Gitterelement also durch ein Verbundmaterial gebildet sein, bei welchem die Kernschicht für eine verbesserte Röntgensichtbarkeit des Implantats sorgt und die Mantelschicht dem Implantat besonders hohe elastische Eigenschaften verleiht. Das superelastische Material kann insbesondere durch ein Formgedächtnismetall gebildet sein, beispielsweise eine Nickel-Titan-Legierung. Derartige Materialien streben danach, eine vorher aufgeprägte Form einzunehmen, wenn das Material eine bestimmte Temperatur erreicht.

Das Gitterelement, insbesondere die Mantelschicht des Gitterelements, kann außerdem eine Titanoxinitridschicht aufweisen, die die Elementoberfläche bildet. Eine solche Titanoxinitridschicht kann durch eine Wärmebehandlung des Implantats in einem Salzbad hergestellt worden. Vorteil einer solchen Titanoxinitridschicht besteht darin, dass sich ein Nickelanteil des Materials der Mantelschicht im Bereich der Innenseite der Titanoxinitridschicht, also der Kernschicht zugewandt, sammelt. An der Außenoberfläche, nämlich der Elementoberfläche, liegt somit kaum Nickel vor, wodurch das sogenannte nickel release reduziert wird. Das nickel release bezeichnet die Fähigkeit von Implantaten, Nickel in den menschlichen Körper abzugeben. Ein möglichst niedriger Abgabewert ist bevorzugt, um allergische Reaktionen zu vermeiden.

Gemäß einer weiteren Ausgestaltung der Erfindung kann vorgesehen sein, dass in die Polymer-Nanostruktur, insbesondere in das Nanovlies, wenigstens ein Medikament eingebettet ist. Geeignete Medikamente können beispielsweise therapeutische Wirkstoffe zur Minimierung einer Restenose, zur Heilung erkrankter Gefäßabschnitte und/oder zur Begrenzung von Zellwachstum umfassen. Andere Medikamente können ebenfalls eingesetzt werden. Indem das Medikament in die Polymer-Nanostruktur eingebettet ist, beispielsweise zwischen den einzelnen Fäden eines Nanovlieses gehalten ist, wird es über einen langen Zeitraum langsam abgegeben und entfaltet so eine langfristige Wirkung.

Im Allgemeinen kann die Gitterstruktur ein superelastisches Material, beispielsweise eine Nickel-Titan-Legierung, wie Nitinol, aufweisen. Die Gitterstruktur kann insbesondere selbstexpandierbar sein. Es ist allerdings auch möglich, dass die Gitterstruktur ein anderes Material, beispielsweise eine Kobalt-Chrom-Legierung umfasst. Die Gitterstruktur kann folglich auch ballonexpandierbar sein. Mit anderen Worten kann eine Expansion der Gitterstruktur durch einen Ballon erfolgen, der beispielweise an einem Katheter angeordnet ist.

Im Rahmen der vorliegenden Anmeldung wird ferner ein Verfahren zur Herstellung eines zuvor beschriebenen Implantats offenbart und beansprucht. Bei dem erfindungsgemäßen Verfahren werden folgende Schritte ausgeführt:
a. Bereitstellen einer Gitterstruktur mit Gitterelementen,
b. Anlegen eines elektrischen Hochspannungsfeldes zwischen der Gitterstruktur und einer Emitterelektrode,
c. Besprühen der Gitterstruktur mit einer Polymerlösung, wobei die Polymerlösung höchstens 3%, insbesondere höchstens 2%, insbesondere höchstens 1%, gelöste Polymeranteile aufweist, und
d. Beschichten der Gitterstruktur mit einer antithrombogenen Beschichtung.

Die Erfindung wird im Folgenden anhand von Ausführungsbeispielen in Bezug auf die beigefügten, schematischen Zeichnungen näher erläutert. Darin zeigen
- Fig. 1: eine Querschnittsansicht eines Gitterelements einer rohrförmigen Gitterstruktur eines erfindungsgemäßen medizinischen Implantats nach einem bevorzugten Ausführungsbeispiel vor dem Aufbringen der Polymer-Nanostruktur;
- Fig. 2: eine Querschnittsansicht des Gitterelements gemäß Fig. 1 nach Aufbringen der Polymer-Nanostruktur;
- Fig. 3: eine Querschnittsansicht eines Gitterelements einer rohrförmigen Gitterstruktur eines erfindungsgemäßen medizinischen Implantats nach einem weiteren bevorzugten Ausführungsbeispiel vor dem Aufbringen der Polymer-Nanostruktur;
- Fig. 4: eine Querschnittsansicht des Gitterelements gemäß Fig. 3 nach Aufbringen der Polymer-Nanostruktur;
- Fig. 5: eine Rasterelektronenmikroskopaufnahme des Gitterelements gemäß Fig. 2;
- Fig. 6: eine Rasterelektronenmikroskopaufnahme des Gitterelements gemäß Fig. 2; und
- Fig. 7: eine Querschnittsansicht eines Polymertröpfchens der Polymer-Nanostruktur des erfindungsgemäßen medizinischen Implantats nach einem bevorzugten Ausführungsbeispiel.

In den beigefügten Zeichnungen sind jeweils Details eines medizinischen Implantats gezeigt, das vorzugsweise als Stent zum Einsatz in Blutgefäßen Verwendung findet. Das Implantat weist eine rohrförmige Gitterstruktur auf, also eine Gitterstruktur, die im Wesentlichen ein Gerüst einer Wandung eines Rohres bildet. Die Rohrform ist also in ihrer Umfangsfläche nicht vollständig geschlossen, sondern weist Gitteröffnungen auf. Generell ist vorgesehen, dass die Gitterstruktur von einem radial komprimierten Zustand in einen radial expandierten Zustand überführbar ist. Das Implantat bzw. dessen Gitterstruktur können also in einer schmalen Bauform durch einen Katheter an den Behandlungsort geführt werden, wobei sich die Gitterstruktur am Behandlungsort entfaltet. Vorzugsweise erfolgt die Entfaltung selbsttätig. Mit anderen Worten ist die Gitterstruktur vorzugsweise selbstexpandierbar.

Die Gitterstruktur umfasst mehrere Gitterelemente 10, die Zellen der Gitterstruktur begrenzen. Die Gitterelemente 10 können durch Stege 11 gebildet sein. Wenn die Gitterelemente 10 durch Stege 11 gebildet sind, ist die Gitterstruktur vorzugsweise durch Schneiden aus einem rohrförmigen Vollmaterial erstellt. Die Stege sind also einstückig miteinander verbunden, so dass die Gitterstruktur insgesamt ein einstückiges Bauteil bildet.

Alternativ können die Gitterelemente 10 auch durch Drähte 12 gebildet sein. Die Drähte 12 sind vorzugsweise miteinander verflochten bzw. verwoben, um so eine Gitterstruktur zu bilden. Die Drähte 12 über- und unterkreuzen sich dabei regelmäßig, wobei das Über- und Unterkreuzungsmuster unterschiedlich sein kann. So kann beispielsweise ein Draht 12 zwei weitere Drähte 12 überkreuzen, um anschließend nur einen Draht 12 zu unterkreuzen. Andere Muster sind denkbar.

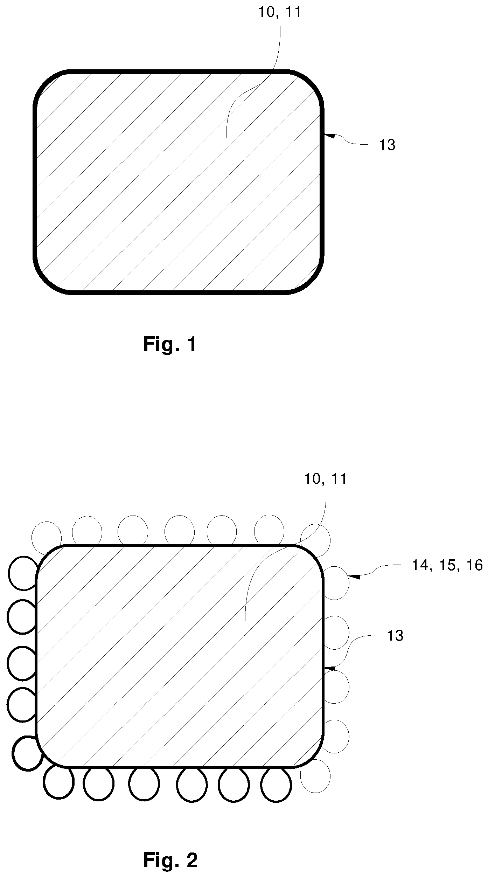

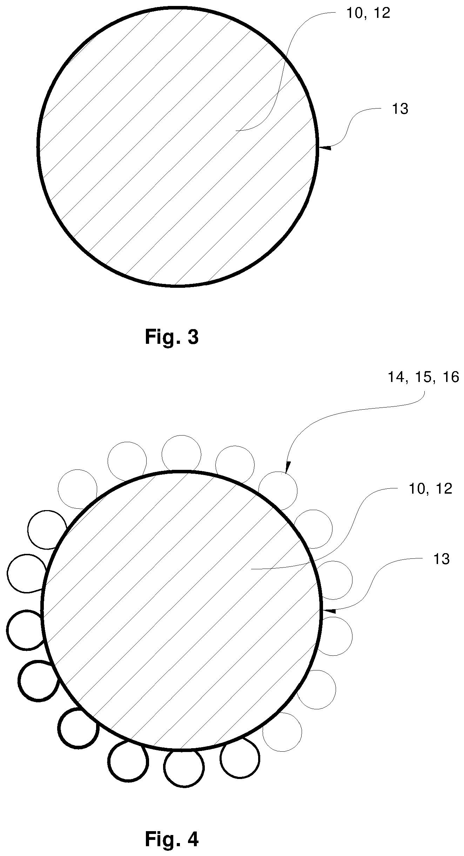

In den Fig. 1 und 2 ist ein Gitterelement 10 gezeigt, das vorzugsweise als Steg 11 ausgebildet ist. Das Gitterelement 10 weist insoweit eine im Wesentlichen rechteckförmige Querschnittskontur mit abgerundeten Kanten auf. Die Abrundung der Kanten kann beispielsweise durch ein Ätzverfahren erzeugt werden. Die Fig. 3 und 4 zeigen hingegen ein Gitterelement 10, das als Draht 12 ausgebildet ist und insoweit einen kreisrunden Querschnitt aufweist.

Die Gitterelemente 10 weisen eine Elementoberfläche 13 auf. Die Elementoberfläche 13 ist bei den Ausführungen gemäß Fig. 1 und 3 im Wesentlichen blank. Es ist erkennbar, dass die Elementoberfläche im Wesentlichen eine glatte Oberfläche bildet. Die Fig. 1 und 3 zeigen also jeweils das Gitterelement 10 im unbeschichteten Zustand.

In Fig. 2 und 4 sind die Gitterelemente 10 gezeigt, die eine Polymer-Nanostruktur 14 aufweisen. Die Polymer-Nanostruktur 14 ist bei diesen Ausführungsbeispielen jeweils durch eine Vielzahl von Polymertröpfchen 15 gebildet. Die Polymertröpfchen 15 können in einem Sprühverfahren auf die Elementoberfläche 13 aufgebracht werden. Es ist erkennbar, dass die Polymer-Nanostruktur 14 bzw. die Polymertröpfchen 15 eine Vergrößerung der Oberfläche des Gitterelements 10 bewirken. Die Polymertröpfchen 15 belegen zwar einen Teil der Elementoberfläche 13, an der sie anhaften. Die Polymertröpfchen 15 selbst weisen jedoch eine zusätzliche, im Wesentlichen kugelsegmentförmige Oberfläche auf, die gemeinsam mit den Lücken zwischen den Polymertröpfchen 15 eine Strukturoberfläche 16 des Gitterelements 10 bilden. Die Strukturoberfläche 16 ist deutlich größer als die Elementoberfläche 13. Insbesondere wird aus der im wesentlichen zweidimensionalen Elementoberfläche 13 durch die Anlagerung der Polymertröpfchen 15 eine dreidimensionale Strukturoberfläche 16 gebildet.

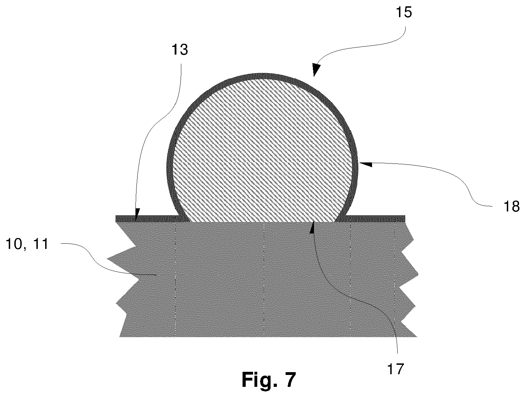

Fig. 7 veranschaulicht das Maß der Oberflächenvergrößerung anhand eines Polymertröpfchens 15. Das Polymertröpfchen 15 haftet, vorzugsweise hydrophob, an der Elementoberfläche 13 des Gitterelements 10 an. Diese (zweidimensionale) Kontaktfläche 17 ist vergleichsweise klein. Das Polymertröpfchen 15 nimmt jedoch eine Form eines (dreidimensionalen) Kugelsegments an, wobei das Kugelsegment größer als eine Halbkugel ist. Die freie Tröpfchenoberfläche 18 ist um ein Vielfaches größer als die Kontaktfläche 17. Auf diese Weise ist es möglich, dass durch Besprühen der Elementoberfläche 13 mit der Polymer-Nanostruktur 14 eine vergrößerte Strukturoberfläche 16 bereitgestellt wird, die um mindestens 50%, insbesondere mindestens 100%, insbesondere mindestens 150%, insbesondere mindestens 200%, insbesondere mindestens 300%, insbesondere mindestens 400%, größer als die Elementoberfläche ist. Mit anderen Worten ist die Strukturoberfläche 16 mindestens anderthalbmal so groß wie die Elementoberfläche 13.

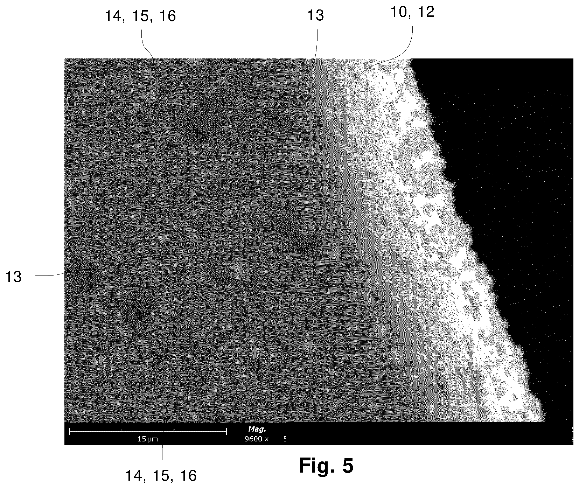

Fig. 5 zeigt eine Rasterelektronenmikroskopaufnahme einer geringfügig ausgeprägten Strukturoberfläche 16 auf dem Gitterelement 10 bzw. Steg 11 gemäß Fig. 2. Es ist erkennbar, dass durch das Aufsprühen der Polymer-Nanostruktur 14 eine im Wesentlichen poröse Haftvermittlerschicht entsteht. Die Porosität ergibt sich dadurch, dass die Polymer-Nanostruktur 14 keine durchgehende, die Elementoberfläche 13 vollständig bedeckende Schicht bildet. Vielmehr ist die Polymer-Nanostruktur 14 aus vielen Polymertröpfchen 15 gebildet, die verteilt auf der Elementoberfläche 13 angeordnet sind. Gerade die verteilte Anordnung der Polymertröpfchen 15 sorgt für die deutliche Vergrößerung der Strukturoberfläche 16 gegenüber der Elementoberfläche 13.

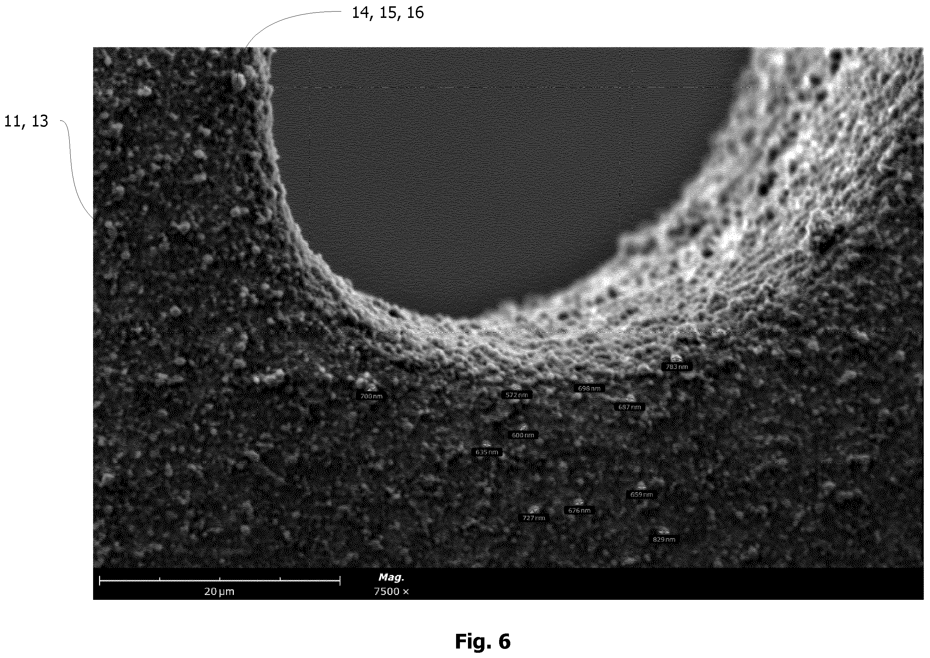

Noch deutlicher ist die Vergrößerung der Strukturoberfläche 16 gegenüber der Elementoberfläche 13, wenn die Dichte der Polymertröpfchen 15 weiter erhöht wird. In Fig. 6 ist eine solche, weitere erhöhte Vergrößerung der Strukturoberfläche 16 gezeigt. Konkret zeigt Fig. 6 eine Rasterelektronenmikroskopaufnahme eines Ausschnitts einer Gitterstruktur aus mehreren Gitterelementen 10, die im vorliegenden Fall als Stege 11 ausgebildet sind. Die Stege 11 sind einstückig miteinander an Verbindungsstellen verbunden. Fig. 6 zeigt einen Teil einer solchen Verbindungsstelle, an welcher zwei Stege 11 winklig aufeinandertreffen bzw. ineinander übergehen. Die Elementoberfläche 13 ist mit der Polymer-Nanostruktur 14 überzogen, die die Elementoberfläche 13 nahezu vollständig bedeckt und die Strukturoberfläche 16 bildet. Die Polymer-Nanostruktur 14 umfasst bzw. besteht aus einer Vielzahl von Polymertröpfchen 15, die durch Aufsprühen einer Polymerlösung auf die Elementoberfläche 13 entstehen können. Die Dichte der Polymertröpfchen 13 ist so hoch gewählt, dass sich zumindest ein Großteil der Polymertröpfchen 13 berühren. Durch die Tröpfchenform der Polymertröpfchen 13 erhöht sich dabei die zur Anhaftung der antithrombogenen Beschichtung verfügbare Haftfläche, die als Strukturoberfläche 16 bezeichnet wird.

Die Polymer-Nanostruktur 14 dient im Wesentlichen als Haftvermittler, so dass eine weitere, in den Zeichnungen nicht dargestellte antithrombogene Nano-Beschichtung gut und in hoher Menge auf dem Gitterelement 10 anhaften kann. Die antithrombogene Beschichtung kann insbesondere eine Kombination aus Fibrin und einem Antikoagulanz enthalten. Besonders wirksame Antikoagulanzien sind Heparin oder Albumin. Eine Kombination von Fibrin und Heparin wird dabei besonders bevorzugt.

Vorteilhaft ist bei der Erfindung nicht nur die reine Vergrößerung der Strukturoberfläche 16 gegenüber der Elementoberfläche 13. Durch die Form und Struktur der Polymertröpfchen 15 kann sich die antithrombogene Beschichtung auch verbessert an der Strukturoberfläche 16 anlagern. Im Wesentlichen entsteht durch die Form der Polymertröpfchen 15, die ein Kugelsegment bilden, welches größer als eine Halbkugel ist, auch eine Bindung zwischen der antithrombogenen Beschichtung und der Strukturoberfläche 16, die zumindest teilweise als formschlüssig bezeichnet werden kann. Der Teil der Tröpfchenoberfläche 18, der die Halbkugelform übergreift und bis zur Kontaktfläche 17 reicht, bildet im Wesentlichen eine Art Hinterschnitt für die Anhaftung der antithrombogenen Beschichtung. Damit ist die antithrombogene Beschichtung besonders langzeitstabil und insbesondere auch resistent gegen Abrieb, der beim Zuführen eines Implantats durch einen Katheter entstehen kann.

Im Ergebnis ist das erfindungsgemäße Implantat also besonders langzeitwirksam und beständig, was zu deutlich verbesserten therapeutischen Erfolgen führt.

Anstelle einer Polymer-Nanostruktur 14, die aus Polymertröpfchen 15 gebildet ist, ist es auch denkbar, die Polymer-Nanostruktur 14 durch ein Nanovlies zu bilden.

Dabei werden Polymermaterialien beispielsweise durch ein Elektrospinnverfahren auf die Elementoberfläche 13 aufgebracht, wobei sich einzelne Fasern chaotisch kreuzen. Zwischen den einzelnen Fasern des Nanovlieses bleibt jedoch ein Luftraum, in welchem sich die antithrombogene Beschichtung anlagern bzw. mit dem polymeren Nanovlies verankern kann. Im Wesentlichen wirkt das Nanovlies also wie ein Schwamm, der die antithrombogene Beschichtung aufsaugt und am Behandlungsort langsam wieder abgibt, so dass die antithrombogene Beschichtung ihre antithrombogene Wirkung langfristig entfalten kann.

### Bezugszeichen

- 10: Gitterelement
- 11: Steg
- 12: Draht
- 13: Elementoberfläche
- 14: Polymer-Nanostruktur
- 15: Polymertröpfchen
- 16: Strukturoberfläche
- 17: Kontaktfläche
- 18: Tröpfchenoberfläche

## Patentansprüche

1. Medizinisches Implantat, insbesondere Stent, mit einer rohrförmigen Gitterstruktur, die von einem radial komprimierten Zustand in einen radial expandierten Zustand überführbar ist und Gitterelemente (10) aufweist, die Zellen der Gitterstruktur begrenzen und eine Elementoberfläche (13) aufweisen, wobei zur Vergrößerung der Elementoberfläche (13) jedes Gitterelements (10) eine Polymer-Nanostruktur (14) vorgesehen ist, die vereinzelt auf der gesamten Elementoberfläche (13) des Gitterelements (10) verteilt anhaftet, wobei eine antithrombogene Beschichtung vorgesehen ist, die sich über die mit der Polymer-Nanostruktur (14) vergrößerte Strukturoberfläche (16) des Gitterelements (10) erstreckt.

2. Implantat nach Anspruch 1,
**dadurch gekennzeichnet, dass**
die Polymer-Nanostruktur (14) hydrophob an die Elementoberfläche (13) gebunden ist.

3. Implantat nach Anspruch 1 oder 2,
**dadurch gekennzeichnet, dass**
die antithrombogene Beschichtung zumindest teilweise in die Polymer-Nanostruktur (14) eingebettet oder mit einer Oberfläche der Polymer-Nanostruktur (14) zumindest teilweise vernetzt ist.

4. Implantat nach einem der vorhergehenden Ansprüche,
**dadurch gekennzeichnet, dass**
die antithrombogene Beschichtung Fibrin und ein Antikoagulanz aufweist.

5. Implantat nach Anspruch 4,
**dadurch gekennzeichnet, dass**
das Antikoagulanz Heparin ist, das kovalent an das Fibrin gebunden ist.

6. Implantat nach einem der vorhergehenden Ansprüche,
**dadurch gekennzeichnet, dass**
die Polymer-Nanostruktur (14) durch Polymertröpfchen (15) gebildet ist, die auf der Elementoberfläche (13) des Gitterelements (10) verteilt angelagert sind.

7. Implantat nach Anspruch 6,
**dadurch gekennzeichnet, dass**
die Polymertröpfchen (15) jeweils einen Durchmesser von höchstens 1500 nm, insbesondere höchstens 1250 nm, insbesondere höchstens 1000 nm, insbesondere höchstens 750 nm, insbesondere höchstens 500 nm, insbesondere höchstens 400 nm, insbesondere höchstens 250 nm, insbesondere höchstens 100 nm, insbesondere höchstens 50 nm, aufweisen.

8. Implantat nach einem der Ansprüche 1 bis 6,
**dadurch gekennzeichnet, dass**
die Polymer-Nanostruktur (14) durch ein Nanovlies aus Polymerfasern gebildet ist, das sich vollständig um das Gitterelement (10) erstreckt.

9. Implantat nach einem der vorhergehenden Ansprüche,
**dadurch gekennzeichnet, dass**
die Strukturoberfläche (16) die Elementoberfläche (13) um mindestens 150%, insbesondere mindestens 200%, insbesondere mindestens 250%, insbesondere mindestens 300%, insbesondere mindestens 400%, insbesondere mindestens 500%, vergrößert.

10. Implantat nach einem der vorhergehenden Ansprüche,
**dadurch gekennzeichnet, dass**
die Elementoberfläche (13) oberflächenbehandelt, insbesondere elektropoliert oder mechanisch blank, ist.

11. Implantat nach einem der vorhergehenden Ansprüche,
**dadurch gekennzeichnet, dass**
das Gitterelement (10) eine Kernschicht und eine Mantelschicht aufweist, wobei die Kernschicht ein röntgensichtbares Material, insbesondere Platin oder eine Platinlegierung, und die Mantelschicht ein superelastisches Metall, insbesondere eine Nickel-Titan-Legierung, aufweist oder daraus besteht.

12. Implantat nach einem der vorhergehenden Ansprüche,
**dadurch gekennzeichnet, dass**
das Gitterelement (10), insbesondere die Mantelschicht, eine Titanoxinitridschicht aufweist, die die Elementoberfläche (13) bildet.

13. Implantat nach einem der vorhergehenden Ansprüche,
**dadurch gekennzeichnet, dass**
in die Polymer-Nanostruktur (14), insbesondere in das Nanovlies, wenigstens ein Medikament, beispielsweise zur Minimierung einer Restenose, zur Heilung erkrankter Gefäßabschnitte und/oder zur Begrenzung von Zellwachstum, eingebettet ist.

14. Verfahren zur Herstellung eines Implantats nach einem der vorhergehenden Ansprüche, bei welchem folgende Schritte ausgeführt werden:
a. Bereitstellen einer Gitterstruktur mit Gitterelementen (10),
b. Anlegen eines elektrischen Hochspannungsfeldes zwischen der Gitterstruktur und einer Emitterelektrode,
c. Besprühen der Gitterstruktur mit einer Polymerlösung, wobei die Polymerlösung höchstens 3%, insbesondere höchstens 2%, insbesondere höchstens 1%, gelöste Polymeranteile aufweist, und
d. Beschichten der Gitterstruktur mit einer antithrombogenen Beschichtung.

## Claims

1. A medical implant, in particular a stent, with a tubular mesh structure which can be transposed from a radially compressed state into a radially expanded state and having mesh elements (10) which delimit cells of the mesh structure and which have an element surface (13),
wherein,
in order to enlarge the element surface (13) of each mesh element (10), a polymeric nanostructure (14) is provided which is distributed over and adhered at isolated points to the entire element surface (13) of the mesh element (10), wherein an antithrombogenic coating is provided which extends over the structural surface (16) of the mesh element (10) enlarged by the polymeric nanostructure (14).

2. The implant as claimed in claim 1,
**characterized in that**
the polymeric nanostructure (14) is hydrophobically bound to the element surface (13).

3. The implant as claimed in claim 1 or claim 2,
**characterized in that**
the antithrombogenic coating is at least partially incorporated into the polymeric nanostructure (14) or is at least partially interlinked with a surface of the polymeric nanostructure (14).

4. The implant as claimed in one of the preceding claims,
**characterized in that**
the antithrombogenic coating contains fibrin and an anticoagulant.

5. The implant as claimed in claim 4,
**characterized in that**
the anticoagulant is heparin which is covalently bound to the fibrin.

6. The implant as claimed in one of the preceding claims,
**characterized in that**
the polymeric nanostructure (14) is formed by polymer droplets (15) which are distributed over and deposited on the element surface (13) of the mesh element (10).

7. The implant as claimed in claim 6,
**characterized in that**
the polymer droplets (15) have a respective diameter of at most 1500 nm, in particular at most 1250 nm, in particular at most 1000 nm, in particular at most 750 nm, in particular at most 500 nm, in particular at most 400 nm, in particular at most 250 nm, in particular at most 100 nm, in particular at most 50 nm.

8. The implant as claimed in one of claims 1 to 6,
**characterized in that**
the polymeric nanostructure (14) is formed by a nano-nonwoven produced from polymer fibres which extends completely around the mesh element (10).

9. The implant as claimed in one of the preceding claims,
**characterized in that**
the structure surface (16) enlarges the element surface (13) by at least 150%, in particular at least 200%, in particular at least 250%, in particular at least 300%, in particular at least 400%, in particular at least 500%.

10. The implant as claimed in one of the preceding claims,
**characterized in that**
the element surface (13) is surface-treated, in particular electropolished or mechanically polished.

11. The implant as claimed in one of the preceding claims,
**characterized in that**
the mesh element (10) has a core layer and a shell layer, wherein the core layer contains or consists of a radiopaque material, in particular platinum or a platinum alloy, and the shell layer contains or consists of a superelastic metal, in particular a nickel-titanium alloy.

12. The implant as claimed in one of the preceding claims, **characterized in that**
the mesh element (10), in particular the shell layer, has a titanium oxynitride layer which forms the element surface (13).

13. The implant as claimed in one of the preceding claims,
**characterized in that**
at least one medicine, for example for minimizing restenosis, for healing diseased vascular sections and/or for limiting cell growth, is incorporated into the polymeric nanostructure (14), in particular into the nano-nonwoven.

14. A method for the production of an implant as claimed in one of the preceding claims, in which the following steps are carried out:
a. providing a mesh structure with mesh elements (10),
b. applying a high-voltage electric field between the mesh structure and an emitter electrode,
c. spraying the mesh structure with a polymer solution, wherein the polymer solution contains at most 3%, in particular at most 2%, in particular at most 1%, of dissolved polymer, and
d. coating the mesh structure with an antithrombogenic coating.

## Revendications

1. Implant médical, en particulier stent, ayant une structure tubulaire en treillis, qui peut être passée d'un état radialement comprimé à un état radialement dilaté, et présente des éléments de grille (10, limitant les cellules de la structure de la grille et présentant une surface d'élément (13),
dans lequel,
pour agrandir la surface d'élément (13) de chaque élément de grille (10), est prévue une nanostructure de polymère (14), qui adhère ponctuellement et de manière répartie sur l'ensemble de la surface d'élément (13) de l'élément de grille (10), étant prévu un revêtement antithrombogène qui s'étend sur la surface structurelle (16) de l'élément de grille (10) agrandie par la nanostructure de polymère (14).

2. Implant selon la revendication 1,
**caractérisé en ce que**
la nanostructure de polymère (14) est liée de manière hydrophobe à la surface d'élément (13).

3. Implant selon la revendication 1 ou 2,
**caractérisé en ce que**
le revêtement antithrombogène est au moins partiellement intégré dans la nanostructure de polymère (14) ou au moins partiellement réticulé avec une surface de la nanostructure de polymère (14).

4. Implant selon l'une des revendications précédentes,
**caractérisé en ce que**
le revêtement antithrombogène présente de la fibrine et un anticoagulant.

5. Implant selon la revendication 4,
**caractérisé en ce que**
l'anticoagulant est l'héparine, qui est liée de manière covalente à la fibrine.

6. Implant selon l'une des revendications précédentes,
**caractérisé en ce que**
la nanostructure de polymère (14) est formée par des gouttelettes de polymère (15) qui sont déposées réparties sur la surface d'élément (13) de l'élément de grille (10).

7. Implant selon la revendication 6,
**caractérisé en ce que**
les gouttelettes de polymère (15) ont chacune un diamètre maximal de 1500 nm, en particulier de 1250 nm, en particulier de 1000 nm, en particulier de 750 nm, en particulier de 500 nm, en particulier de 400 nm, en particulier de 250 nm, en particulier de 100 nm, et en particulier de 50 nm.

8. Implant selon l'une des revendications 1 à 6, **caractérisé en ce que**
la nanostructure de polymère (14) est formée par une nano-couche de fibres de polymère qui s'étend entièrement autour de l'élément de grille (10).

9. Implant selon l'une des revendications précédentes,
**caractérisé en ce que**
la surface structurelle (16) agrandit la surface de l'élément (13) d'au moins 150 %, en particulier au moins 200 %, en particulier au moins 250 %, en particulier au moins 300 %, en particulier au moins 400 %, en particulier au moins 500 %.

10. Implant selon l'une des revendications précédentes,
**caractérisé en ce que**
la surface de l'élément (13) est traitée en surface, en particulier électropolie ou est mécaniquement nue.

11. Implant selon l'une des revendications précédentes,
**caractérisé en ce que**
l'élément de grille (10) a une couche de noyau et une couche de gaine, la couche de noyau comportant un matériau visible aux rayons X, en particulier du platine ou un alliage de platine, et que la couche de gaine est un métal super-élastique, en particulier un alliage de nickel-titane, ou est composée de celui-ci.

12. Implant selon l'une des revendications précédentes,
**caractérisé en ce que**
l'élément de grille (10), en particulier la couche de gaine, présente une couche de nitrure d'oxyde de titane qui forme la surface de l'élément (13).

13. Implant selon l'une des revendications précédentes,
**caractérisé en ce que**
la nanostructure de polymère (14), en particulier la nano-couche, contient au moins un médicament, par exemple pour minimiser la resténose, guérir les sections des vaisseaux malades et/ou limiter la croissance cellulaire.

14. Procédé de fabrication d'un implant selon l'une des revendications précédentes, dans lequel les étapes suivantes sont effectuées :
a. prévision d'une structure de grille avec des éléments de grille (10),
b. application d'un champ électrique à haute tension entre la structure de la grille et une électrode émettrice ;
c. pulvérisation de la structure de la grille avec une solution de polymère, la solution de polymère présentant au plus 3 %, en particulier au plus 2 %, en particulier au plus 1 % de parts de polymère dissoutes, et
d. revêtement de la structure de la grille avec un revêtement antithrombogène.
